Europäisches Patentamt

European Patent Office

Office européen des brevets

(11) Veröffentlichungsnummer: **0 339 530**
**A2**

## EUROPÄISCHE PATENTANMELDUNG

(21) Anmeldenummer: **89107313.2**

(51) Int. Cl.⁴: **A61F 2/36**

(22) Anmeldetag: **22.04.89**

(30) Priorität: **26.04.88 DE 3813944**

(43) Veröffentlichungstag der Anmeldung:
**02.11.89 Patentblatt 89/44**

(84) Benannte Vertragsstaaten:
**AT BE CH DE ES FR GB GR IT LI LU NL SE**

(71) Anmelder: **S + G IMPLANTS GMBH**
**Grapengiesserstrasse 34**
**D-2400 Lübeck(DE)**

(72) Erfinder: **Grundei, Hans**
**Hamburger Strasse 89**
**D-2400 Lübeck(DE)**
Erfinder: **Poppenbütteler, Wolfram Thomas,**
**Prof. Dr.**
**Landstrasse 12**
**D-2000 Hamburg 65(DE)**

(74) Vertreter: **Wilcken, Thomas, Dipl.-Ing. et al**
**Musterbahn 1**
**D-2400 Lübeck(DE)**

(54) **Endoprothese für ein Gelenk.**

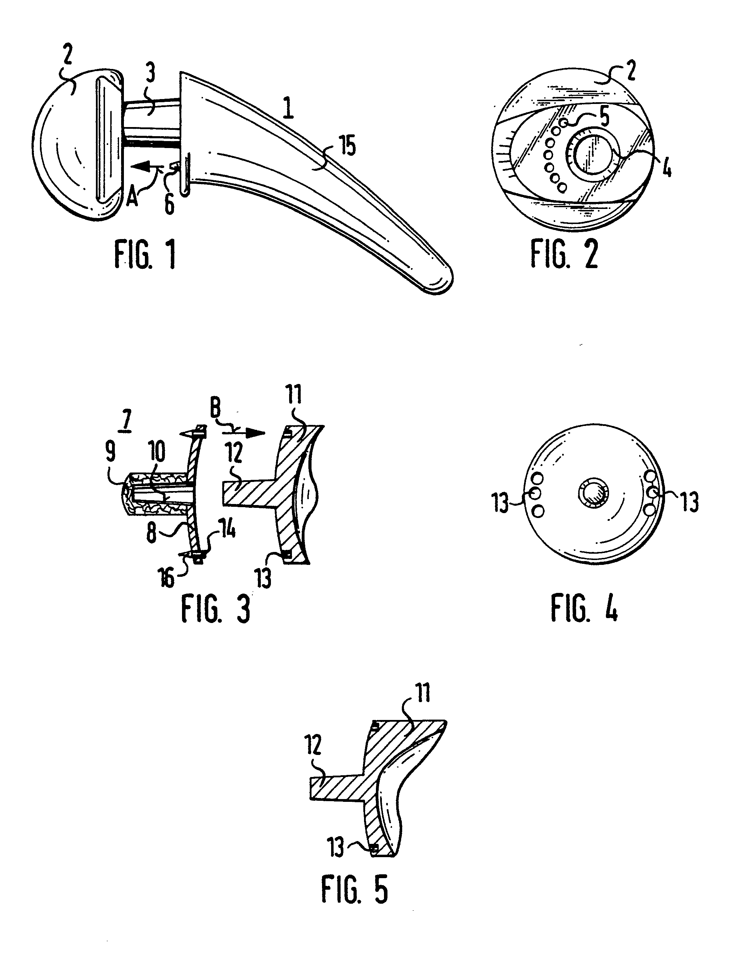

(57) Endoprothese für ein Gelenk, bestehend aus einem Anschlußstück (1) mit einem in einem Röhrenknochen fixierbaren Stiel, einem darauf sitzenden Gelenkkopf (2) und/oder einem eine Gelenkpfanne nachbildenden Gelenkpfannenteil, wobei die Rotationsstellung des Gelenkkopfes (2) am Anschlußstück (1) und/oder des Gelenkpfannenteils einstellbar und fixierbar ist (Figuren 1 und 2).

FIG. 1

FIG. 2

## Endoprothese für ein Gelenk

Die Erfindung betrifft eine Endoprothese für ein Gelenk nach dem Oberbegriff des Anspruchs 1.

Derartige Prothesen bestehen im allgemeinen aus einem Anschlußstück mit einem Stiel, welcher in den Markraum eines resezierten Röhrenknochens einführbar ist und dort fixierbar ist, beispielsweise unter Anwendung von Zement.

Bekannte Gelenkendoprothesen tragen am distalen Ende einen Gelenkkopf, der entweder fest oder mittels einer konischen Steckverbindung form- und kraftschlüssig mit dem Stiel verbunden ist.

Bei einer vorgegebenen Form des in den Markraum des Knochens zu führenden Stiels des Anschlußstückes ergeben sich aufgrund der natürlichen individuellen Ausformung des Markraumes Probleme hinsichtlich der Stellung des Gelenkkopfes in bezug auf die Gelenkpfanne. So erfährt das Anschlußstück aufgrund einer Quasi-Torsion des Knochens vom distalen zum proximalen Ende, welche Größenordnungen bis zu 45° aufweisen können, beim Eintreiben des Stiel in den Markraum eine Drehung. Aufgrund einer gegebenen Ovalität des Gelenkkopfes ist ein optimales Zusammenarbeiten des Gelenkkopfes mit der Gelenkpfanne nach dem Einbau des Anschlußstückes dann nicht mehr gewährleistet.

Der Erfindung liegt nun die Aufgabe zugrunde, eine Endoprothese für ein Gelenk der eingangs genannten Art so weiterzubilden, daß eine individuelle Einstellung der Gelenkstellung trotz vorgegebener Form des in den Markraum des Röhrenknochens einzuführenden Stiels möglich ist.

Die Aufgabe wird gelöst durch eine Endoprothese mit den Merkmalen des Anspruchs 1.

Weitere vorteilhafte Ausgestaltungen ergeben sich aus den Unteransprüchen.

Gemäß diesem Lösungsvorschlag wird eine aus einem Anschlußstück mit einem in einem Röhrenknochen fixierbaren Stiel und einem konischen Steckzapfen, der mit einer konischen Steckhülse eines auf das Anschlußstück setzbaren Gelenkkopfes in eine selbsthemmende Verbindung bringbar ist, und aus einem am knöcherigen Gegenstück des zu ersetzenden Gelenkes anbringbaren Gelenkpfannenteil bestehende Endoprothese so ausgebildet, daß die Rotationsstellung des Gelenkkopfes auf dem Konus des Anschlußstückes einstellbar und fixierbar ist.

Gemäß diesem Vorschlag ist es möglich, nach dem Eintreiben des Stiels des Anschlußstückes in den Markraum des Röhrenknochens, wobei dieser unvermeidlich der natürlichen individuellen Ausformung des Markraumes folgt, den Gelenkkopf auf den Steckkonus des Anschlußstückes zu setzen, dessen Lage optimal durch Verdrehen auf dem Steckkonus einzustellen und in dieser zu fixieren.

Vorzugsweise erfolgt die Fixierung der Rotationsstellung mittels konzentrisch um die konische Steckhülse des Gelenkkopfes angeordnete Bohrungen und durch einen Fixierzapfen an der Stirnseite des Anschlußstückes, wobei der Fixierzapfen in eine der Bohrungen greift, so daß der Fixierzapfen die Winkelstellung des Gelenkkopfes auf dem Anschlußstück arretiert. Nach der Einstellung der Rotationsstellung des Gelenkkopfes wird die selbsthemmende Verbindung zwischen der konischen Steckhülse im Gelenkkopf und dem konischen Zapfen am Anschlußstück hergestellt, so daß der Gelenkkopf belastungsfest in seiner Lage gehalten wird.

Gemäß einer weiteren vorteilhaften Ausbildung der erfindungsgemäßen Prothese ist aber nicht nur der Gelenkkopf am Anschlußstück in seiner Lage einstellbar und fixierbar, sondern auch das Gelenkpfannenteil. Diese Maßnahme vergrößert die Bandbreite der Möglichkeiten von Gelenkstellungen erheblich, so daß eine optimale Einstellung des Gelenkes in Anpassung anatomischer Gegebenheiten gewährleistet ist.

Dazu besteht das Gelenkpfannenteil vorzugsweise aus einer an den Knochen anbringbaren Unterlage mit einem zentralen Zapfen, welcher in den Knochen getrieben wird und in dem eine konische Steckhülse ausgebildet ist, sowie aus einem eine Gelenkpfanne nachbildenden Adaptionsteil, welches einen konischen Steckzapfen aufweist. Die Verbindung zwischen der Unterlage und dem Adaptionsteil erfolgt dabei über eine selbsthemmende Konusverbindung zwischen dem konischen Steckzapfen der Unterlage und dem Steckzapfen des Adaptionsteils.

Vor der Herstellung der selbsthemmenden Konusverbindung kann die Rotationsstellung des Adaptionsteils auf der Unterlage eingestellt werden, und zwar durch einfaches Drehen um die Achse seines konischen Steckzapfens. Die Fixierung der Rotationsstellung kann mittels konzentrisch um den Steckzapfen des Adaptionsteils angeordnete Bohrungen einerseits und durch mindestens einen Fixierzapfen an der Stirnseite der Unterlage andererseits erfolgen, wobei der Fixierzapfen in eine der Bohrungen greift.

Nach Einstellung der Rotationsstellung und ihrer Fixierung mittels des Fixierzapfens wird die selbsthemmende Konusverbindung hergestellt, so daß das Adaptionsteil belastungsfest in Lage gehalten wird.

Die Fixierung der Unterlage des Gelenkpfannenteils am Knochen kann durch mindestens einen in den Knochen treibbaren Zapfen an der Unterseite der Unterlage erfolgen.

Das mit dem Gelenkkopf nach Einbau der Prothese zusammenarbeitende Teil des Gelenkpfannenteils , also das Adaptionsteil, besteht für eine optimale Funktion der Prothese aus einem Kunststoff mit niedrigem Reibungskoeffizienten.

Für eine zementlose Fixation des Stieles des Anschlußstückes im Röhrenknochen und des zentralen Zapfens der Unterlage des Gelenkpfannenteils können deren Oberflächenstruktur den spongiösen Knochen nachgebildet sein.

Die Erfindung wird anhand der Zeichnungen näher erläutert. Hierbei zeigt:

Figur 1 eine Seitenansicht eines Anschlußstückes der Endoprothese mit Gelenkkopf,

Figur 2 eine Ansicht auf die Unterseite des Gelenkkopfes in Richtung des Pfeiles A in Fig. 1,

Figur 3 eine seitliche Schnittansicht des Gelenkpfannenteils mit Unterlage und Adaptionsteil,

Figur 4 eine Ansicht auf die Unterseite des Adaptionsteils des Gelenkpfannenteils in Richtung des Pfeiles B in Fig. 3, und

Figur 5 eine Schnittansicht einer weiteren Ausführungsform des Adaptionsteils des Gelenkpfannenteils.

Gemäß Figur 1 besteht das Anschlußstück 1 aus einem in einem Röhrenknochen fixierbaren Stiel 15. Am gelenkseitigen Ende weist das Anschlußstück 15 einen konischen Steckzapfen 3 auf. Auf den konischen Steckzapfen 3 ist ein Gelenkkopf 2 aufsteckbar. Hierzu weist der Gelenkkopf 2 eine konische Steckhülse 4 auf. Die Verbindung zwischen dem Gelenkkopf 2 und dem Anschlußstück 1 erfolgt über eine selbsthemmende Konusverbindung zwischen dem Steckzapfen 3 und der Steckhülse 4.

Wie in Fig. 2 dargestellt, weist der Gelenkkopf 2 an seiner Unterseite konzentrisch um die konische Steckhülse 4 angeordnete Bohrungen 5 auf. Nach dem Aufsetzen des Gelenkkopfes 2 auf den konischen Steckzapfen 3 des Anschlußstückes 1 kann dessen Stellung durch Verdrehen auf dem Steckzapfen 3 eingestellt werden und durch ein Eingreifen eines Fixierzapfens 6 an der Stirnseite des Anschlußstückes 1 in eine der Bohrungen 5 im Gelenkkopf 2 fixiert werden. Nach Herstellung der selbsthemmenden Konusverbindung zwischen Gelenkkopf 2 und dem Anschlußstück 1 wird der Gelenkkopf belastungsfest in seiner Lage auf dem Anschlußstück 1 gehalten.

Das Gelenkpfannenteil gemäß Figur 3 besteht aus einer Unterlage, die am Knochen befestigt wird, und einem Adaptionsteil 11. Die Unterlage 8 weist einen zentralen Zapfen 9 auf, der in den Knochen eingesetzt wird. Im Inneren des zentralen Zapfens 9 ist eine konische Steckhülse 10 vorgesehen. Das Adaptionsteil 11 weist einen konischen Steckzapfen 12 auf, der in die konische Steckhülse 10 der Unterlage 8 steckbar ist. Vor dem Herstellen einer selbsthemmenden Konusverbindung zwischen dem Steckzapfen 12 des Adaptionsteils 11 und der konischen Steckhülse 10 im zentralen Zapfen 9 der Unterlage 8 kann das Adaptionsteil 11 um den Steckzapfen 12 verdreht werden, um die Stellung des als Gelenkpfanne ausgebildeten Adaptionsteils 11 zu justieren.

Gemäß Figur 4 weist das Adaptionsteil 11 an seiner Unterseite konzentrisch um den Steckzapfen 12 angeordnete Bohrungen 13 zur Fixierung seiner Stellung in bezug auf die Unterlage 8 auf.

Die Fixierung der Stellung des Adaptionsteils 11 in bezug auf die Unterlage 8 erfolgt durch ein Eingreifen mindestens eines Fixierzapfens 14 an der Stirnseite der Unterlage 8, von denen in Figur 3 zwei Stück dargestellt sind. Nach Herstellung der selbsthemmenden Konusverbindung zwischen der Unterlage 8 und dem Adaptionsteil 11 wird dieses in der justierten Lage belastungsfest gehalten.

Für die Fixierung der Unterlage 8 am Knochen weist diese an seiner dem Knochen zugewandten Seite mindestens einen in den Knochen treibbaren Zapfen 16 auf, von denen in Figur 3 zwei Stück dargestellt sind.

In der dargestellten Ausführungsform der Unterlage 8 bildet der Fixierzapfen 14 mit dem Zapfen 16 eine bauliche Einheit in Form eines Stiftes.

In Figur 5 ist ein etwas abgewandeltes Ausführungsbeispiel für das Adaptionsteil 11 dargestellt. In Abweichung von dem in Figur 3 dargestellten Adaptionsteil weist dessen Ausführungsform gemäß Figur 5 eine andere Gestaltung der eigentlichen Gelenkpfanne auf, und zwar mit einer dachförmigen Auswölbung, welche den Gelenkkopf weiter umschließt.

In an sich bekannter Weise ist das die eigentliche Gelenkpfanne darstellende Adaptionsteil 11 aus einem Kunststoff mit niedrigem Reibungskoeffizienten gefertigt.

Wie in Figur 1 und Figur 3 angedeutet, sind die Oberflächenstrukturen des Stiels 15 des Anschlußstückes 1 und des zentralen Zapfens 9 der Unterlage 8 dem spongiösen Knochen nachgebildet , so daß die Fixation der Endoprothese im Knochen vorteilhafterweise zementlos erfolgen kann.

## Ansprüche

1. Endoprothese für ein Gelenk, bestehend aus einem Anschlußstück mit einem in einem Röhrenknochen fixierbaren Stiel und einem konischen Steckzapfen , der mit einer konischen Steckhülse eines auf das Anschlußstück setzbaren Gelenkkopfes in eine selbsthemmende Verbindung bringbar ist und aus einem am knöcherigen Gegenstück des zu ersetzenden Gelenkes anbringbaren Gelenkpfannenteil,
dadurch gekennzeichnet, daß die Rotationsstellung des Gelenkkopfes (2) auf den Konus(3) des Anschlußstückes (1) einstellbar und fixierbar ist.

2. Endoprothese nach Anspruch 1, dadurch gekennzeichnet,daß die Fixierung der Rotationsstellung des Gelenkkopfes (2) auf dem Anschlußstück (1) mittels konzentrisch um die konische Steckhülse (4) des Gelenkkopfes (2) angeordnete Bohrungen (5) einerseits und durch einen Fixierzapfen (6) an der Stirnseite des Anschlußstückes (1) andererseits erfolgt, wobei der Fixierzapfen (6) in eine der Bohrungen (5) greift.

3. Endoprothese nach einem der Ansprüche 1 bis 2, dadurch gekennzeichnet, daß das Gelenkpfannenteil (7) aus einer an den Knochen anbringbaren Unterlage (8) mit einem zentralen Zapfen (9) , in dem eine konische Steckhülse (10) ausgebildet ist, und aus einem eine Gelenkpfanne nachbildenden Adaptionsteil (11)besteht, das einen konischen Steckzapfen (12) aufweist, der in eine selbsthemmende Klemmverbindung mit der konischen Steckhülse(10) der Unterlage (8) bringbar ist.

4. Endoprothese nach Anspruch 3, dadurch gekennzeichnet, daß die Rotationsstellung des Adaptionsteils (11) auf der Unterlage (8) einstellbar und fixierbar ist.

5. Endoprothese nach Anspruch 4, dadurch gekennzeichnet, daß die Fixierung der Rotationsstellung des Adaptionsteils (11) auf der Unterlage (8) mittels konzentrisch um seinen Steckzapfen (12) angeordnete Bohrungen (13) einerseits und durch mindestens einen Fixierzapfen(14) an der Stirnseite der Unterlage (8) andererseits erfolgt, wobei der Fixierzapfen (14) in eine der Bohrungen (13) greift.

6. Endoprothese nach einem der Ansprüche 3 bis 5, dadurch gekennzeichnet, daß die Unterlage (8) des Gelenkpfannenteils(7) mittels mindestens eines in den Knochen treibbaren Zapfens(16) an ihrer Unterseite am Knochen fixierbar ist.

7. Endoprothese nach einem der Ansprüche 1 bis 6, dadurch gekennzeichnet, daß das mit dem Gelenkkopf (2) nach Einbau zusammenarbeitende Teil des Gelenkpfannenteils (7) aus einem Kunststoff mit niedrigem Reibungskoeffizienten besteht.

8. Endoprothese nach einem der Ansprüche 1 bis 7, dadurch gekennzeichnet, daß die Oberflächenstruktur des Stiels (15) des Anschlußstückes (1) dem spongiösen Knochen nachgebildet ist.

9. Endoprothese nach einem der Ansprüche 3 bis 8, dadurch gekennzeichnet, daß die Oberflächenstruktur des zentralen Zapfens(9) der Unterlage (8)dem spongiösen Knochen nachgebildet ist.

FIG. 1

FIG. 2

FIG. 3

FIG. 4

FIG. 5